(19) Europäisches Patentamt
European Patent Office
Office européen des brevets

(11) **EP 4 631 435 A1**

(12) **EUROPEAN PATENT APPLICATION**

(43) Date of publication:
**15.10.2025  Bulletin 2025/42**

(21) Application number: **25168148.2**

(22) Date of filing: **03.04.2025**

(51) International Patent Classification (IPC):
**A61B 6/03** (2006.01)    **A61B 6/42** (2024.01)
**A61B 6/00** (2024.01)

(52) Cooperative Patent Classification (CPC):
**A61B 6/03; A61B 6/4241; A61B 6/5217; A61B 6/54**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **08.04.2024  US 202418629023**

(71) Applicant: **Siemens Healthineers AG**
**91301 Forchheim (DE)**

(72) Inventors:
• **ZHANG, Yanbo**
**Plainsboro, NJ 08536 (US)**
• **ADIYOSO, Arnaud Arindra**
**90431 Nürnberg (DE)**

• **GEORGESCU, Bogdan**
**Princeton, NJ 08540 (US)**
• **BALACHANDRAN, Abishek**
**600010 Chennai, Tamil Nadu (IN)**
• **GEIGER, Bernhard**
**Cranbury, NJ 08512 (US)**
• **GHESU, Florin-Cristian**
**91083 Baiersdorf (DE)**
• **GRBIC, Sasa**
**Plainsboro, NJ 08536 (US)**
• **COMANICIU, Dorin**
**Princeton, NJ 08540 (US)**

(74) Representative: **HKW Intellectual Property PartG mbB**
**Theresienhöhe 12**
**80339 München (DE)**

(54) **COMPUTER-AIDED DIAGNOSIS SYSTEM FOR PULMONARY NODULE ANALYSIS USING PCCT IMAGES**

(57)    Systems and methods for performing one or more medical imaging analysis tasks on PCCT (photon-counting computed tomography) images are provided. Image acquisition parameters of a PCCT image acquisition device are determined for acquiring PCCT images. One or more PCCT images of an anatomical object of a patient acquired using the PCCT image acquisition device configured with the image acquisition parameters are received. One or more medical imaging analysis tasks analyzing the anatomical object are performed based on the one or more PCCT images using one or more machine learning based models. Results of the one or more medical imaging analysis tasks are output.

FIG. 2

200

Determine image acquisition parameters of a PCCT image acquisition device for acquiring PCCT images
202

Receive one or more PCCT images of an anatomical object of a patient acquired using the PCCT image acquisition device configured with the image acquisition parameters
204

Perform one or more medical imaging analysis tasks analyzing the anatomical object based on the one or more PCCT images using one or more machine learning based models
206

Output results of the one or more medical imaging analysis tasks
208

EP 4 631 435 A1

**Description**

TECHNICAL FIELD

The present invention relates generally to computer-aided diagnosis, and in particular to computer-aided diagnosis for pulmonary nodule analysis using PCCT (photon-counting computed tomography) images.

BACKGROUND

In the current clinical workflow, computer-aided diagnosis systems are utilized for detecting pulmonary nodules from chest CT (computed tomography) images, thereby mitigating lung cancer mortality. Recently, PCCT (photon-counting CT) imaging has been introduced, in which x-rays are detected using a photon-counting detector to register the interactions of individual photons and keep track of the spectrum of deposited energy in each interaction. As compared with traditional CT imaging, PCCT imaging offers higher resolution and spectral information. However, conventional computer-aided diagnosis systems are unable to exploit the advantages of PCCT imaging.

BRIEF SUMMARY OF THE INVENTION

In accordance with one or more embodiments, systems and methods for computer-aided diagnosis of pulmonary nodules using PCCT imaging are provided.

In one embodiment, systems and methods for performing one or more medical imaging analysis tasks on PCCT (photon-counting computed tomography) images are provided. Image acquisition parameters of a PCCT image acquisition device are determined for acquiring PCCT images. One or more PCCT images of an anatomical object of a patient acquired using the PCCT image acquisition device configured with the image acquisition parameters are received. One or more medical imaging analysis tasks analyzing the anatomical object are performed based on the one or more PCCT images using one or more machine learning based models. Results of the one or more medical imaging analysis tasks are output.

In one embodiment, a plurality of candidate PCCT images is acquired using varying image acquisition parameters. The plurality of candidate PCCT images is presented to a user. Input is received from the user selecting one of the plurality of candidate PCCT images. The image acquisition parameters are determined as parameters corresponding to the selected candidate PCCT images.

In one embodiment, a plurality of candidate PCCT images are acquired using varying image acquisition parameters. One of the plurality of candidate PCCT images is identified as having a highest analytical accuracy for performing the one or more medical imaging analysis tasks using the one or more machine learning based models. The image acquisition parameters are determined as parameters corresponding to the identified candidate PCCT images.

In one embodiment, the image acquisition parameters of the PCCT image acquisition device are determined for acquiring PCCT images optimized for performing the one or more medical imaging analysis tasks.

In one embodiment, the image acquisition parameters comprise a number of energy bands and associated energy thresholds. In one embodiment, the image acquisition parameters comprise at least one of reconstructed image spacing, slice thickness, reconstruction kernels, or dose.

In one embodiment, the one or more medical imaging analysis tasks comprise at least one of detection, segmentation, size quantification, typology classification, or malignancy assessment of the anatomical object of the patient.

In one embodiment, the one or more machine learning based models are trained using annotated PCCT training images.

In one embodiment, the anatomical object comprises a pulmonary nodule of the patient.

These and other advantages of the invention will be apparent to those of ordinary skill in the art by reference to the following detailed description and the accompanying drawings.

BRIEF DESCRIPTION OF THE DRAWINGS

Figure 1 shows a workflow for performing one or more medical imaging analysis tasks analyzing a pulmonary nodule, in accordance with one or more embodiments;

Figure 2 shows a method for performing one or more medical imaging analysis tasks analyzing an anatomical object, in accordance with one or more embodiments;

Figure 3 shows an exemplary artificial neural network that may be used to implement one or more embodiments;

Figure 4 shows a convolutional neural network that may be used to implement one or more embodiments;

Figure 5 shows a data flow diagram for using a generative adversarial network, in accordance with one or more embodiments;

Figure 6 shows a schematic structure of a recurrent machine learning model that may be used to implement one or more embodiments; and

Figure 7 shows a high-level block diagram of a computer that may be used to implement one or more embodiments.

DETAILED DESCRIPTION

The present invention generally relates to methods and systems for CAD (computer-aided diagnosis) system for pulmonary nodule analysis using PCCT (photon-counting computed tomography) imaging. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system. Further, reference herein to pixels of an image may refer equally to voxels of an image and vice versa.

Embodiments described herein provide for a computer-aided diagnosis system for pulmonary nodule analysis from PCCT imaging data. The pulmonary nodule analysis comprises two principal modules: 1) an imaging configuration module for determining image acquisition parameters of a PCCT image acquisition device and 2) a nodule analysis module for performing one or more medical imaging analysis tasks on one or more PCCT images acquired using the PCCT image acquisition device configured with the image acquisition parameters. The imaging configuration module and the nodule analysis module work together synergistically to optimize nodule detection and analysis. Advantageously, embodiments of the present invention provide a tailored design for PCCT, ensuring greater compatibility and precision in PCCT environments. Further, embodiments of the present invention provide for optimized image acquisition parameters, ensuring that the resulting PCCT images are best suited for nodule analysis tasks. In addition, embodiments of the present invention provide superior nodule analysis by leveraging the high-resolution and spectral advantages of PCCT to provide, for example, enhanced detection of nodules smaller than 3 millimeters in diameter and improved nodule type and malignancy classification.

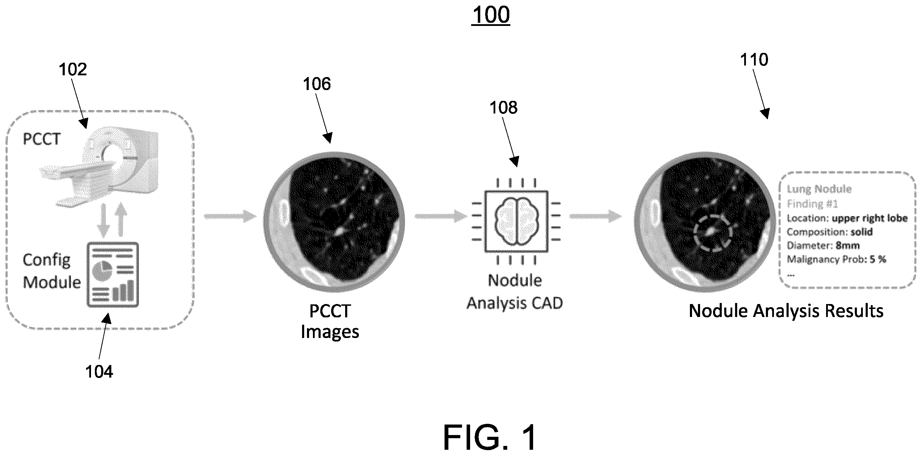

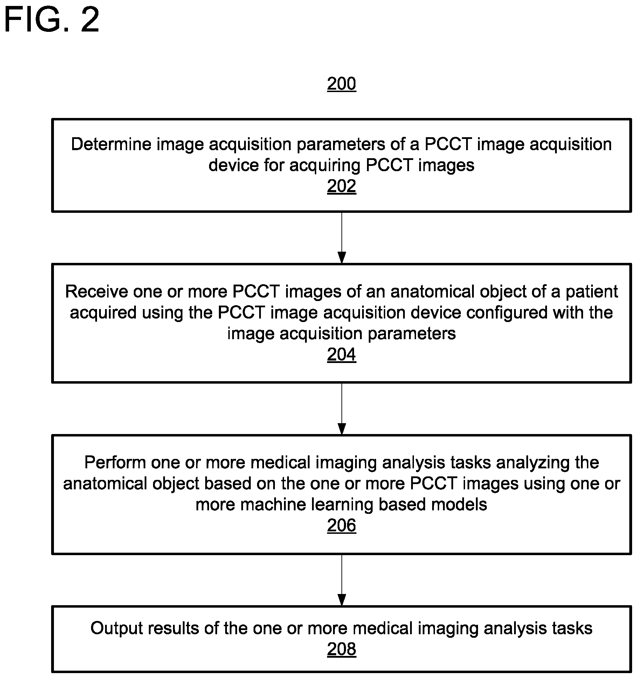

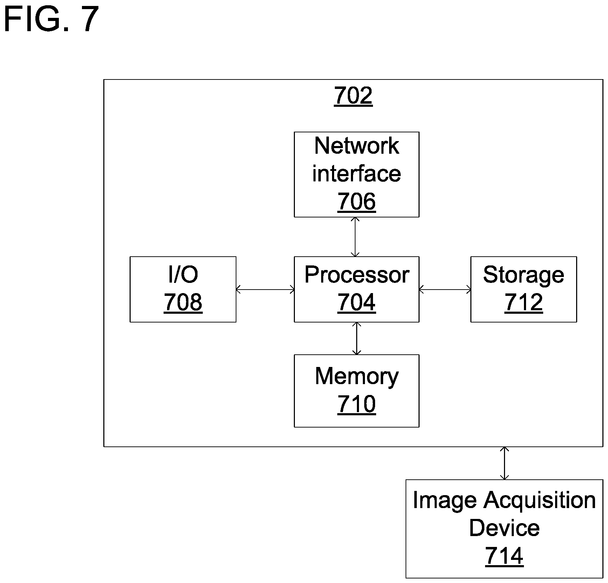

Figure 1 shows a workflow 100 for performing one or more medical imaging analysis tasks analyzing a pulmonary nodule, in accordance with one or more embodiments. Figure 2 shows a method 200 for performing one or more medical imaging analysis tasks analyzing an anatomical object, in accordance with one or more embodiments. The steps of method 200 may be performed by one or more suitable computing devices, such as, e.g., computer 702 of Figure 7. Figure 1 and Figure 2 will be described together.

At step 202 of Figure 2, image acquisition parameters of a PCCT image acquisition device are determined for acquiring PCCT images. The image acquisition parameters may be determined by an imaging configuration module, which may be integrated within the PCCT image acquisition device to ensure PCCT image acquisitions are tailor-made for analysis. In one example, as shown in workflow 100 of Figure 1 configuration module 104 determines image acquisition parameters of PCCT image acquisition device 102. The image acquisition parameters are determined to acquire PCCT images optimized for performing one or more medical imaging analysis tasks (e.g., at step 206 of Figure 2).

In one embodiment, the image acquisition parameters comprise the number of energy bands and their associated energy thresholds, with and without administration of a contrast agent. In PCCT imaging, PCCT detectors resolve the incident x-ray energy spectrum into multiple energy bands or bins (e.g., 2 to 8 energy bands). The energy bands allow for differentiation between tissue types and contrast agents. Each energy band corresponds to a specific range of x-ray energy, enabling more accurate characterization. The PCCT detectors measure energy deposited by each x-ray photon as an electric pulse proportional to the energy. Pulse heights are compared with the energy threshold that reflects a specified photon energy level. By setting different energy thresholds, the incoming x-ray photons are sorted into the defined energy bands. However, the image acquisition parameters may comprise any other suitable parameter for acquiring PCCT images, such as, e.g., reconstructed image spacing, slice thickness, reconstruction kernels, and/or dose.

In one embodiment, the image acquisition parameters are determined according to a subjective evaluation. In this embodiment, a plurality of candidate PCCT images of one or more different patients are acquired using varying image acquisition parameters. The image acquisition parameters may be varied, for example, by starting with default image acquisition parameters for the PCCT image acquisition device and iteratively modifying one or more parameters. The plurality of candidate PCCT images are presented to a user, such as, e.g., a radiologist (e.g., via a display device of a computing system) and input is received from the user selecting one of the plurality of candidate PCCT images that provides the optimal image for performing the one or more medical imaging analysis tasks (e.g., nodule detections and analysis). The image acquisition parameters are determined as the image acquisition parameters corresponding to the selected candidate PCCT image.

In one embodiment, the image acquisition parameters are determined according to an objective evaluation. In this embodiment, similar to the subjective evaluation, a plurality of candidate PCCT images of one or more different patients are acquired using varying image acquisition parameters. An analysis system utilizing one or more machine learning based models is applied to the plurality of candidate PCCT images to identify one of the plurality of candidate PCCT images with a highest analytical accuracy for performing the one or more medical imaging analysis tasks. The analysis system utilizing the one or more machine learning based models is the same as applied at step 208 of Figure 2, described in further detail below. The image acquisition parameters are determined as the image acquisition parameters corresponding to the identified candidate PCCT image.

In one embodiment, step 202 of Figure 2 is not performed and method 200 starts at step 204 using predetermined (e.g., default) image acquisition parameters.

At step 204 of Figure 2, one or more PCCT images of an anatomical object of a patient acquired using the PCCT image acquisition device configured with the image acquisition parameters are received. In one example, as shown in workflow 100 of Figure 1, the one or more PCCT images are PCCT images 106. In one embodiment, the anatomical object is a nodule (e.g., a pulmonary nodule). However, the anatomical object may be any other suitable object of interest of the patient, such as, e.g., organs, vessels, bones, other types of abnormalities, etc.

The one or more PCCT images may be received, for example, by directly receiving the one or more PCCT images from the PCCT image acquisition device (e.g., image acquisition device 714 of Figure 7) as the PCCT images are acquired, by loading the one or more PCCT images from a storage or memory of a computer system (e.g., storage 712 or memory 710 of computer 702 of Figure 7), or by receiving the one or more PCCT images from a remote computer system (e.g., computer 702 of Figure 7). Such a computer system or remote computer system may comprise one or more patient databases, such as, e.g. , an EHR (electronic health record), EMR (electronic medical record), PHR (personal health record), HIS (health information system), RIS (radiology information system), PACS (picture archiving and communication system), LIMS (laboratory information management system), or any other suitable database or system.

At step 206 of Figure 2, one or more medical imaging analysis tasks analyzing the anatomical object are performed based on the one or more PCCT images using one or more machine learning based models. The one or more medical imaging analysis tasks may be performed by an analysis module. In one example, as shown in workflow 100 of Figure 1, one or more medical imaging analysis tasks for analyzing a nodule are performed by nodule analysis CAD module 108 based on PCCT images 106 to generate nodule analysis results 110.

In one embodiment, the one or more medical imaging analysis tasks comprise detection, segmentation, size quantification, typology classification, and malignancy assessment of the anatomical object of the patient. However, the one or more medical imaging analysis tasks may comprise any other suitable task for analyzing the anatomical object. The one or more medical imaging analysis tasks may be performed using any suitable machine learning based models (e.g., well-known machine learning based models). The machine learning based models receive as input the one or more PCCT images and generates as output results of the one or more medical imaging analysis tasks.

The one or more machine learning based models are trained to perform the one or more medical imaging analysis tasks during a prior offline or training stage using PCCT training images. Due to the higher spatial resolution and spatial information provided by the PCCT training images, the PCCT training images may be annotated with higher quality labels as compared to labels for conventional CT images. Once trained, the one or more machine learning based models are applied during an online or inference stage, e.g., to perform step 206 of Figure 2.

The one or more machine learning based models trained with PCCT training images provides several advantages. In one example, the machine learning models trained with PCCT training images may provide enhanced small nodule detection. Annotators can more accurately label nodules smaller than 3 millimeters in diameter due to the high resolution of PCCT images, thereby improving the performance of the machine learning based models for detecting small nodules (i.e., less than 3 millimeters in diameter) and identification of early-stage cancer. In another example, the machine learning models trained with PCCT training images may provide advanced classification capabilities. The PCCT training images provide precise and stable CT numbers (i.e., the pixel values) as well as additional spectral information, thereby enhancing the performance of the machine learning based models for nodule typology and malignancy classification.

At step 208 of Figure 2, results of the one or more medical imaging analysis tasks are output. For example, the results of the one or more medical imaging analysis tasks can be output by displaying the results on a display device of a computer system (e.g., I/O 708 of computer 702 of Figure 7), storing the results on a memory or storage of a computer system (e.g., memory 710 or storage 712 of computer 702 of Figure 7), or by transmitting the results to a remote computer system (e.g., computer 702 of Figure 7).

Embodiments described herein are described with respect to the claimed systems as well as with respect to the claimed methods. Features, advantages or alternative embodiments herein can be assigned to the other claimed objects and vice versa. In other words, claims and embodiments for the systems can be improved with features described or claimed in the context of the respective methods. In this case, the functional features of the method are implemented by physical units of the system.

Furthermore, certain embodiments described herein are described with respect to methods and systems utilizing trained machine learning models, as well as with respect to methods and systems for providing trained machine learning models. Features, advantages or alternative embodiments herein can be assigned to the other claimed objects and vice versa. In other words, claims and embodiments for providing trained machine learning models can be improved with features described or claimed in the context of utilizing trained machine learning models, and vice versa. In particular, datasets used in the methods and systems for utilizing trained machine learning models can have the same properties and features as the corresponding datasets used in the methods and systems for providing trained machine learning models, and the trained machine learning models provided by the respective methods and systems can be used in the methods and systems for utilizing the trained machine learning models.

In general, a trained machine learning model mimics cognitive functions that humans associate with other human minds. In particular, by training based on training data the machine learning model is able to adapt to new circumstances and to detect and extrapolate patterns. Another term for "trained machine learning model" is "trained function."

In general, parameters of a machine learning model can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine learning models can be adapted iteratively by several steps of training. In particular, within the training a certain cost function can be minimized. In particular, within the training of a neural network the backpropagation algorithm can be used.

In particular, machine learning models disclosed herein, such as, e.g., the one or more machine learning based models utilized by nodule analysis CAD module 108 of Figure 1 of the one or more machine learning based models utilized at step 206 of Figure 2, can comprise, for example, a neural network, a support vector machine, a decision tree and/or a Bayesian network, and/or the machine learning model can be based on, for example, k-means clustering, Q-learning, genetic algorithms and/or association rules. In particular, a neural network can be, e.g., a deep neural network, a convolutional neural network or a convolutional deep neural network. Furthermore, a neural network can be, e.g., an adversarial network, a deep adversarial network and/or a generative adversarial network.

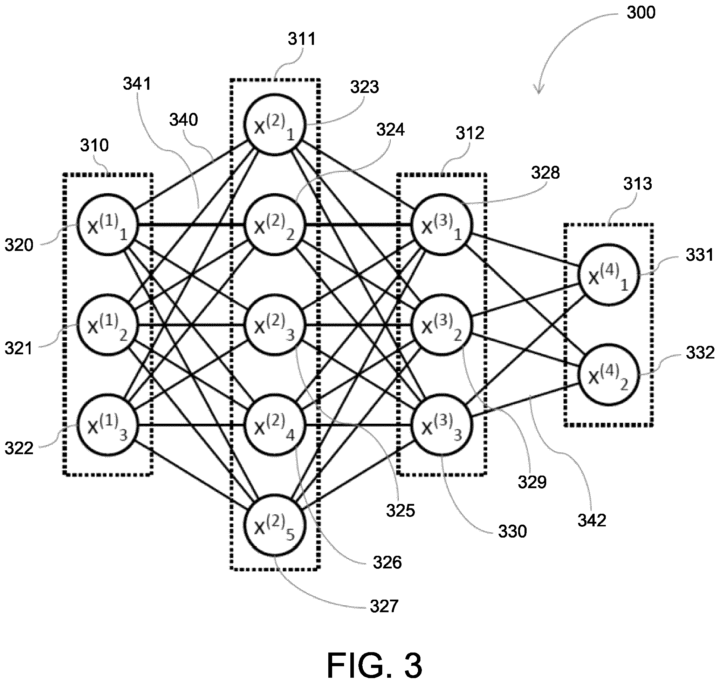

Figure 3 shows an embodiment of an artificial neural network 300 that may be used to implement one or more machine learning models described herein. Alternative terms for "artificial neural network" are "neural network", "artificial neural net" or "neural net".

The artificial neural network 300 comprises nodes 320, ..., 332 and edges 340, ..., 342, wherein each edge 340, ..., 342 is a directed connection from a first node 320, ..., 332 to a second node 320, ..., 332. In general, the first node 320, ..., 332 and the second node 320, ..., 332 are different nodes 320, ..., 332, it is also possible that the first node 320, ..., 332 and the second node 320, ..., 332 are identical. For example, in Figure 3 the edge 340 is a directed connection from the node 320 to the node 323, and the edge 342 is a directed connection from the node 330 to the node 332. An edge 340, ..., 342 from a first node 320, ..., 332 to a second node 320, ..., 332 is also denoted as "ingoing edge" for the second node 320, ..., 332 and as "outgoing edge" for the first node 320, ..., 332.

In this embodiment, the nodes 320, ..., 332 of the artificial neural network 300 can be arranged in layers 310, ..., 313, wherein the layers can comprise an intrinsic order introduced by the edges 340, ..., 342 between the nodes 320, ..., 332. In particular, edges 340, ..., 342 can exist only between neighboring layers of nodes. In the displayed embodiment, there is an input layer 310 comprising only nodes 320, ..., 322 without an incoming edge, an output layer 313 comprising only nodes 331, 332 without outgoing edges, and hidden layers 311, 312 in-between the input layer 310 and the output layer 313. In general, the number of hidden layers 311, 312 can be chosen arbitrarily. The number of nodes 320, ..., 322 within the input layer 310 usually relates to the number of input values of the neural network, and the number of nodes 331, 332 within the output layer 313 usually relates to the number of output values of the neural network.

In particular, a (real) number can be assigned as a value to every node 320, ..., 332 of the neural network 300. Here, $x^{(n)}_i$ denotes the value of the i-th node 320, ..., 332 of the n-th layer 310, ..., 313. The values of the nodes 320, ..., 322 of the input layer 310 are equivalent to the input values of the neural network 300, the values of the nodes 331, 332 of the output layer 313 are equivalent to the output value of the neural network 300. Furthermore, each edge 340, ..., 342 can comprise a weight being a real number, in particular, the weight is a real number within the interval [-1, 1] or within the interval [0, 1]. Here, $w^{(m,n)}_{i,j}$ denotes the weight of the edge between the i-th node 320, ..., 332 of the m-th layer 310, ..., 313 and the j-th node 320, ..., 332 of the n-th layer 310, ..., 313. Furthermore, the abbreviation $w^{(n)}_{i,j}$ is defined for the weight $w^{(n, n+1)}_{i,j}$.

In particular, to calculate the output values of the neural network 300, the input values are propagated through the neural network. In particular, the values of the nodes 320, ..., 332 of the (n+1)-th layer 310, ..., 313 can be calculated based on the values of the nodes 320, ..., 332 of the n-th layer 310, ..., 313 by

$$x^{(n+1)_j} = f\left(\sum_i x^{(n)_i} \cdot w^{(n)_{i,j}}\right).$$

Herein, the function f is a transfer function (another term is "activation function"). Known transfer functions are step functions, sigmoid function (e.g., the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 310 are given by the input of the neural network 300, wherein values of the first hid-den layer 311 can be calculated based on the values of the input layer 310 of the neural network, wherein values of the second hidden layer 312 can be calculated based in the values of the first hidden layer 311, etc.

In order to set the values $w^{(m, n)}_{i,j}$ for the edges, the neural network 300 has to be trained using training data. In particular, training data comprises training input data and training output data (denoted as $t_i$). For a training step, the neural network 300 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 300 (backpropagation algorithm). In particular, the weights are changed according to

$$w'^{(n)_{i,j}} = w^{(n)_{i,j}} - \gamma \cdot \delta^{(n)_j} \cdot x^{(n)_i}$$

wherein y is a learning rate, and the numbers $\delta^{(n)}_j$ can be recursively calculated as

$$\delta^{(n)_j} = \left(\sum_k \delta^{(n+1)_k} \cdot w^{(n+1)_{j,k}}\right) \cdot f'\left(\sum_i x^{(n)_i} \cdot w^{(n)_{i,j}}\right)$$

based on $\delta^{(n+1)}_j$, if the (n+1)-th layer is not the output layer, and

$$\delta^{(n)_j} = \left(x^{(n+1)_j} - t^{(n+1)_j}\right) \cdot f'\left(x^{(n)_i} \cdot w^{(n)_{i,j}}\right)$$

if the (n+1)-th layer is the output layer 313, wherein f is the first derivative of the activation function, and $t^{(n+1)}_j$ is the comparison training value for the j-th node of the output layer 313.

A convolutional neural network is a neural network that uses a convolution operation instead general matrix multiplication in at least one of its layers (so-called "convolutional layer"). In particular, a convolutional layer performs a dot product of one or more convolution kernels with the convolutional layer's input data / image, wherein the entries of the one or more convolution kernel are the parameters or weights that are adapted by training. In particular, one can use the Frobenius inner product and the ReLU activation function. A convolutional neural network can comprise additional layers, e.g., pooling layers, fully connected layers, and normalization layers.

By using convolutional neural networks input images can be processed in a very efficient way, because a convolution operation based on different kernels can extract various image features, so that by adapting the weights of the convolution kernel the relevant image features can be found during training. Furthermore, based on the weight-sharing in the convolutional kernels less parameters need to be trained, which prevents overfitting in the training phase and allows to have faster training or more layers in the network, improving the performance of the network.

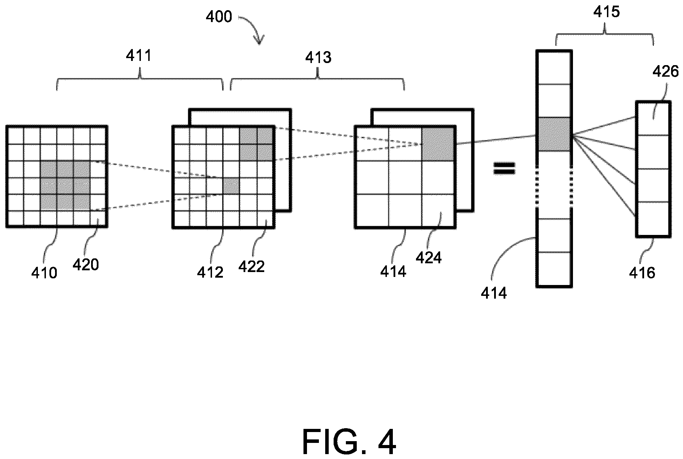

Figure 4 shows an embodiment of a convolutional neural network 400 that may be used to implement one or more machine learning models described herein. In the displayed embodiment, the convolutional neural network comprises 400 an input node layer 410, a convolutional layer 411, a pooling layer 413, a fully connected layer 414 and an output node layer 416, as well as hidden node layers 412, 414. Alternatively, the convolutional neural network 400 can comprise several convolutional layers 411, several pooling layers 413 and several fully connected layers 415, as well as other types of layers. The order of the layers can be chosen arbitrarily, usually fully connected layers 415 are used as the last layers before the output layer 416.

In particular, within a convolutional neural network 400 nodes 420, 422, 424 of a node layer 410, 412, 414 can be considered to be arranged as a d-dimensional matrix or as a d-dimensional image. In particular, in the two-dimensional case the value of the node 420, 422, 424 indexed with i and j in the n-th node layer 410, 412, 414 can be denoted as x(n)[i, j]. However, the arrangement of the nodes 420, 422, 424 of one node layer 410, 412, 414 does not have an effect on the calculations executed within the convolutional neural network 400 as such, since these are given solely by the structure and the weights of the edges.

A convolutional layer 411 is a connection layer between an anterior node layer 410 (with node values x(n-1)) and a posterior node layer 412 (with node values x(n)). In particular, a convolutional layer 411 is characterized by the structure and the weights of the incoming edges forming a convolution operation based on a certain number of kernels. In particular, the structure and the weights of the edges of the convolutional layer 411 are chosen such that the values x(n) of the nodes 422 of the posterior node layer 412 are calculated as a convolution x(n) = K * x(n-1) based on the values x(n-1) of the nodes 420 anterior node layer 410, where the convolution * is defined in the two-dimensional case as

$$x_k^{(n)}[i,j] = \left(K * x^{(n-1)}\right)[i,j] = \sum_{i'}\sum_{j'} K\,[i',j'] \cdot x^{(n-1)}[i-i',\, j-j'].$$

Here the kernel K is a d-dimensional matrix (in this embodiment, a two-dimensional matrix), which is usually small compared to the number of nodes 420, 422 (e.g., a 3x3 matrix, or a 5x5 matrix). In particular, this implies that the weights of the edges in the convolution layer 411 are not independent, but chosen such that they produce said convolution equation. In particular, for a kernel being a 3x3 matrix, there are only 9 independent weights (each entry of the kernel matrix corresponding to one independent weight), irrespectively of the number of nodes 420, 422 in the anterior node layer 410 and the posterior node layer 412.

In general, convolutional neural networks 400 use node layers 410, 412, 414 with a plurality of channels, in particular, due to the use of a plurality of kernels in convolutional layers 411. In those cases, the node layers can be considered as (d+1)-dimensional matrices (the first dimension indexing the channels). The action of a convolutional layer 411 is then a two-dimensional example defined as

$$x^{(n)_b}[i,j] = \sum_a K_{a,b} * x^{(n-1)_a}[i,j] = \sum_a \sum_{i'}\sum_{j'} K_{a,b}[i',j'] \cdot x^{(n-1)_a}[i-i',j-j']$$

where $x^{(n-1)}{}_a$ corresponds to the a-th channel of the anterior node layer 410, $x^{(n)}{}_b$ corresponds to the b-th channel of the posterior node layer 412 and $K_{a,b}$ corresponds to one of the kernels. If a convolutional layer 411 acts on an anterior node layer 410 with A channels and outputs a posterior node layer 412 with B channels, there are A·B independent d-dimensional kernels $K_{a,b}$.

In general, in convolutional neural networks 400 activation functions are used. In this embodiment re ReLU (acronym for "Rectified Linear Units") is used, with R(z) = max(0, z), so that the action of the convolutional layer 411 in the two-dimensional example is

$$x^{(n)_b}[i,j] = R\left(\sum_a \left(K_{a,b} * x^{(n-1)a}\right)[i,j]\right) = R\left(\sum_a \sum_{i'}\sum_{j'} K_{a,b}[i',j'] \cdot x^{(n-1)a}[i-i',j-j']\right)$$

It is also possible to use other activation functions, e.g., ELU (acronym for "Exponential Linear Unit"), LeakyReLU, Sigmoid, Tanh or Softmax.

In the displayed embodiment, the input layer 410 comprises 36 nodes 420, arranged as a two-dimensional 6x6 matrix. The first hidden node layer 412 comprises 72 nodes 422, arranged as two two-dimensional 6x6 matrices, each of the two matrices being the result of a convolution of the values of the input layer with a 3x3 kernel within the convolutional layer 411. Equivalently, the nodes 422 of the first hidden node layer 412 can be interpreted as arranged as a three-dimensional 2x6x6 matrix, wherein the first dimension correspond to the channel dimension.

The advantage of using convolutional layers 411 is that spatially local correlation of the input data can exploited by enforcing a local connectivity pattern between nodes of adjacent layers, in particular by each node being connected to only a small region of the nodes of the preceding layer.

A pooling layer 413 is a connection layer between an anterior node layer 412 (with node values x(n-1)) and a posterior node layer 414 (with node values x(n)). In particular, a pooling layer 413 can be characterized by the structure and the weights of the edges and the activation function forming a pooling operation based on a nonlinear pooling function f. For example, in the two-dimensional case the values x(n) of the nodes 424 of the posterior node layer 414 can be calculated based on the values x(n-1) of the nodes 422 of the anterior node layer 412 as

$$x^{(n)_b}[i,j] = f(x^{(n-1)}[id_1, jd_2], \ldots, x^{(n-1)_b}[(i+1)d_1 - 1, (j+1)d_2 - 1])$$

In other words, by using a pooling layer 413 the number of nodes 422, 424 can be reduced, by re-placing a number d1 ·d2 of neighboring nodes 422 in the anterior node layer 412 with a single node 422 in the posterior node layer 414 being calculated as a function of the values of said number of neighboring nodes. In particular, the pooling function f can be the max-function, the average or the L2-Norm. In particular, for a pooling layer 413 the weights of the incoming edges are fixed and are not modified by training.

The advantage of using a pooling layer 413 is that the number of nodes 422, 424 and the number of parameters is reduced. This leads to the amount of computation in the network being reduced and to a control of overfitting.

In the displayed embodiment, the pooling layer 413 is a max-pooling layer, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

In general, the last layers of a convolutional neural network 400 are fully connected layers 415. A fully connected layer 415 is a connection layer between an anterior node layer 414 and a posterior node layer 416. A fully connected layer 413 can be characterized by the fact that a majority, in particular, all edges between nodes 414 of the anterior node layer 414 and the nodes 416 of the posterior node layer are present, and wherein the weight of each of these edges can be adjusted individually.

In this embodiment, the nodes 424 of the anterior node layer 414 of the fully connected layer 415 are displayed both as two-dimensional matrices, and additionally as non-related nodes (indicated as a line of nodes, wherein the number of nodes was reduced for a better presentability). This operation is also denoted as "flattening". In this embodiment, the number of nodes 426 in the posterior node layer 416 of the fully connected layer 415 smaller than the number of nodes 424 in the anterior node layer 414. Alternatively, the number of nodes 426 can be equal or larger.

Furthermore, in this embodiment the Softmax activation function is used within the fully connected layer 415. By applying the Softmax function, the sum the values of all nodes 426 of the output layer 416 is 1, and all values of all nodes 426 of the output layer 416 are real numbers between 0 and 1. In particular, if using the convolutional neural network 400 for categorizing input data, the values of the output layer 416 can be interpreted as the probability of the input data falling into one of the different categories.

In particular, convolutional neural networks 400 can be trained based on the backpropagation algorithm. For preventing overfitting, methods of regularization can be used, e.g., dropout of nodes 420, ..., 424, stochastic pooling, use of artificial data, weight decay based on the L1 or the L2 norm, or max norm constraints.

According to an aspect, the machine learning model may comprise one or more residual networks (ResNet). In particular, a ResNet is an artificial neural network comprising at least one jump or skip connection used to jump over at least one layer of the artificial neural network. In particular, a ResNet may be a convolutional neural network comprising one or more skip connections respectively skipping one or more convolutional layers. According to some examples, the ResNets may be represented as m-layer ResNets, where m is the number of layers in the corresponding architecture and, according to some examples, may take values of 34, 50, 101, or 152. According to some examples, such an m-layer ResNet may respectively comprise (m-2)/2 skip connections.

A skip connection may be seen as a bypass which directly feeds the output of one preceding layer over one or more bypassed layers to a layer succeeding the one or more bypassed layers. Instead of having to directly fit a desired mapping, the bypassed layers would then have to fit a residual mapping "balancing" the directly fed output.

Fitting the residual mapping is computationally easier to optimize than the directed mapping. What is more, this alleviates the problem of vanishing/exploding gradients during optimization upon training the machine learning models: if a bypassed layer runs into such problems, its contribution may be skipped by regularization of the directly fed output. Using ResNets thus brings about the advantage that much deeper networks may be trained.

A generative adversarial model (an acronym is GA model) comprises a generative function and a discriminative function, wherein the generative function creates synthetic data, and the discriminative function distinguishes between synthetic and real data. By training the generative function and/or the discriminative function on the one hand the generative function is configured to create synthetic data which is incorrectly classified by the discriminative function as real, on the other hand the discriminative function is configured to distinguish between real data and synthetic data generated by the generative function. In the notion of game theory, a generative adversarial model can be interpreted as a zero-sum game. The training of the generative function and/or of the discriminative function is based, in particular, on the minimization of a cost function.

By using a GA model, based on a set of training data synthetic data can be generated that has the same characteristics as the training data set. The training of the GA model can be based on data not being annotated (unsupervised learning), so that there is low effort in training a GA model.

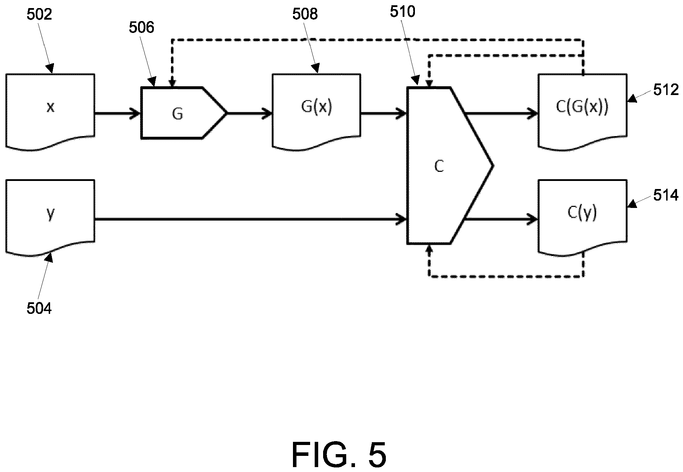

Figure 5 shows a data flow diagram according to an embodiment for using a generative adversarial network for creating synthetic output data G(x) 508 based on input data x 502 that is indistinguishable from real output data y 504, in accordance with one or more embodiments. The synthetic output data G(x) 508 has the same structure as the real output data y 504, but its content is not derived from real world data.

The generative adversarial network comprises a generator function G 506 and a classifier function C 510 which are trained jointly. The task of the generator function G 506 is to provide realistic synthetic output data G(x) 508 based on input data x 502, and the task of the classifier function C 510 is to distinguish between real output data y 504 and synthetic output data G(x) 508. In particular, the output of the classifier function C 510 is a real number between 0 and 1 corresponding to the probability of the input value being real data, so that an ideal classifier function would calculate an output value of $C(y)$ 514 $\approx$ 1 for real data y 504 and $C(G(x))$ 512 $\approx$ 0 for synthetic data G(x) 508.

Within the training process, parameters of the generator function G 506 are adapted so that the synthetic output data G(x) 508 has the same characteristics as real output data y 504, so that the classifier function C 510 cannot distinguish between real and synthetic data anymore. At the same time, parameters of the classifier function C 510 are adapted so that it distinguishes between real and synthetic data in the best possible way. Here, the training relies on pairs comprising input data x 502 and the corresponding real output data y 504. Within a single training step, the generator function G 506 is applied to the input data x 502 for generating synthetic output data G(x) 508. Furthermore, the classifier function C 510 is applied to the real output data y 504 for generating a first classification result C(y) 514. Additionally, the classifier function C 510 is applied to the synthetic output data G(x) 508 for generating a second classification result C(G(x)) 512.

Adapting the parameters of the generative function G 506 and the classifier function C 510 is based on minimizing a cost function by using the backpropagation algorithm, respectively. In this embodiment, the cost function $K_C$ for the classifier function C 510 is $K_C \propto - BCE(C(y), 1) - BCE(C(G(x)), 0)$, wherein BCE denotes the binary cross entropy defined as $BCE(z, z') = z' \cdot \log(z) + (1 - z') \cdot \log(1 - z)$. By using this cost function, both wrongly classifying real output data as synthetic (indicated by $C(y) \approx 0$) and wrongly classifying synthetic output data as real (indicated as $C(G(x))$ 512 $\approx$ 1) increases the cost function $K_C$ to be minimized. Furthermore, the cost function $K_G$ for the generator function G 506 is $K_G \propto - BCE(C(G(x)), 1) = - \log(C(G(x))$. By using this cost function, correctly classified synthetic output data (indicated as $C(G(x))$ 512 $\approx$ 0) leads to an increase of the cost function $K_G$ to be minimized.

In particular, a recurrent machine learning model is a machine learning model whose output does not only depend on the input value and the parameters of the machine learning model adapted by the training process, but also on a hidden state vector, wherein the hidden state vector is based on previous inputs used on for the recurrent machine learning model. In particular, the recurrent machine learning model can comprise additional storage states or additional structures that incorporate time delays or comprise feedback loops.

In particular, the underlying structure of a recurrent machine learning model can be a neural network, which can be denoted as recurrent neural network. Such a recurrent neural network can be described as an artificial neural network where connections between nodes form a directed graph along a temporal sequence. In particular, a recurrent neural network can be interpreted as directed acyclic graph. In particular, the recurrent neural network can be a finite impulse recurrent neural network or an infinite impulse recurrent neural network (wherein a finite impulse network can be unrolled and replaced with a strictly feedforward neural network, and an infinite impulse network cannot be unrolled and replaced with a strictly feedforward neural network).

In particular, training a recurrent neural network can be based on the BPTT algorithm (acronym for "backpropagation through time"), on the RTRL algorithm (acronym for "real-time recurrent learning") and/or on genetic algorithms.

By using a recurrent machine learning model input data comprising sequences of variable length can be used. In particular, this implies that the method cannot be used only for a fixed number of input datasets (and needs to be trained differently for every other number of input datasets used as input), but can be used for an arbitrary number of input datasets. This implies that the whole set of training data, independent of the number of input datasets contained in different sequences, can be used within the training, and that training data is not reduced to training data corresponding to a certain number of successive input datasets.

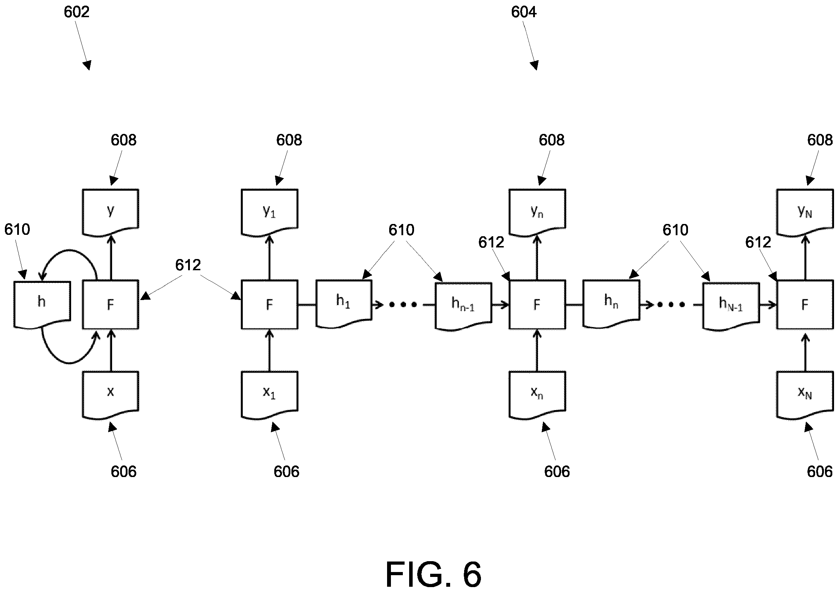

Figure 6 shows the schematic structure of a recurrent machine learning model F, both in a recurrent representation 602 and in an unfolded representation 604, that may be used to implement one or more machine learning models described herein. The recurrent machine learning model takes as input several input datasets $x, x_1, ..., x_N$ 606 and creates a corresponding set of output datasets $y, y_1, ..., y_N$ 608. Furthermore, the output depends on a so-called hidden vector $h, h_1, ..., h_N$ 610, which implicitly comprises information about input datasets previously used as input for the recurrent machine learning model F 612. By using these hidden vectors $h, h_1, ..., h_N$ 610, a sequentiality of the input datasets can be leveraged.

In a single step of the processing, the recurrent machine learning model F 612 takes as input the hidden vector $h_{n-1}$ created within the previous step and an input dataset $x_n$. Within this step, the recurrent machine learning model F generates as output an updated hidden vector $h_n$ and an output dataset $y_n$. In other words, one step of processing calculates $(y_n, h_n) = F(x_n, h_{n-1})$, or by splitting the recurrent machine learning model F 612 into a part F(y) calculating the output data and F(h) calculating the hidden vector, one step of processing calculates $y_n = F^{(y)}(x_n, h_{n-1})$ and $h_n = F^{(h)}(x_n, h_{n-1})$. For the first processing step, $h_0$ can be chosen randomly or filled with all entries being zero. The parameters of the recurrent machine learning model F 612 that were trained based on training datasets before do not change between the different processing steps.

In particular, the output data and the hidden vector of a processing step depend on all the previous input datasets used in the previous steps. $y_n = F^{(y)}(x_n, F^{(h)}(x_{n-1}, h_{n-2}))$ and $h_n = F(h)(x_n, F^{(h)}(x_{n-1}, h_{n-2}))$.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatuses, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatuses, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 1 or 2. Certain steps or functions of the methods and workflows described herein, including one or more of the steps or functions of Figures 1 or 2, may be performed by a server or by another processor in a network-based cloud-computing system. Certain steps or functions of the methods and workflows described herein, including one or more of the steps of Figures 1 or 2, may be performed by a client computer in a network-based cloud computing system. The steps or functions of the methods and workflows described herein, including one or more of the steps of Figures 1 or 2, may be performed by a server and/or by a client computer in a network-based cloud computing system, in any combination.

Systems, apparatuses, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 1 or 2, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 702 that may be used to implement systems, apparatuses, and methods described herein is depicted in Figure 7. Computer 702 includes a processor 704 operatively coupled to a data storage device 712 and a memory 710. Processor 704 controls the overall operation of computer 702 by executing computer program instructions that define such operations. The computer program instructions may be stored in data storage device 712, or other computer readable medium, and loaded into memory 710 when execution of the computer program instructions is desired. Thus, the method and workflow steps or functions of Figures 1 or 2 can be defined by the computer program instructions stored in memory 710 and/or data storage device 712 and controlled by processor 704 executing the computer program instructions. For example, the computer program instructions can be implemented as computer executable code programmed by one skilled in the art to perform the method and workflow steps or functions of Figures 1 or 2. Accordingly, by executing the computer program instructions, the processor 704 executes the method and workflow steps or functions of Figures 1 or 2. Computer 702 may also include one or more network interfaces 706 for communicating with other devices via a network. Computer 702 may also include one or more input/output devices 708 that enable user interaction with computer 702 (e.g., display, keyboard, mouse, speakers, buttons, etc.).

Processor 704 may include both general and special purpose microprocessors, and may be the sole processor or one of multiple processors of computer 702. Processor 704 may include one or more central processing units (CPUs), for example. Processor 704, data storage device 712, and/or memory 710 may include, be supplemented by, or incorporated in, one or more application-specific integrated circuits (ASICs) and/or one or more field programmable gate arrays (FPGAs).

Data storage device 712 and memory 710 each include a tangible non-transitory computer readable storage medium. Data storage device 712, and memory 710, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 708 may include peripherals, such as a printer, scanner, display screen, etc. For example, input/output devices 708 may include a display device such as a cathode ray tube (CRT) or liquid crystal display (LCD) monitor for displaying information to the user, a keyboard, and a pointing device such as a mouse or a trackball by which the user can provide input to computer 702.

An image acquisition device 714 can be connected to the computer 702 to input image data (e.g., medical images) to the computer 702. It is possible to implement the image acquisition device 714 and the computer 702 as one device. It is also possible that the image acquisition device 714 and the computer 702 communicate wirelessly through a network. In a possible embodiment, the computer 702 can be located remotely with respect to the image acquisition device 714.

Any or all of the systems, apparatuses, and methods discussed herein may be implemented using one or more computers such as computer 702.

One skilled in the art will recognize that an implementation of an actual computer or computer system may have other structures and may contain other components as well, and that Figure 7 is a high level representation of some of the components of such a computer for illustrative purposes.

Independent of the grammatical term usage, individuals with male, female or other gender identities are included within the term.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope of the invention. Those skilled in the art could implement various other feature combinations without departing from the scope of the invention.

The following is a list of non-limiting illustrative embodiments disclosed herein:

Illustrative embodiment 1. A computer-implemented method comprising: determining image acquisition parameters of a PCCT (photon-counting computed tomography) image acquisition device for acquiring PCCT images; receiving one or more PCCT images of an anatomical object of a patient acquired using the PCCT image acquisition device configured with the image acquisition parameters; performing one or more medical imaging analysis tasks analyzing the anatomical object based on the one or more PCCT images using one or more machine learning based models; and outputting results of the one or more medical imaging analysis tasks.

Illustrative embodiment 2. The computer-implemented method of illustrative embodiment 1, wherein determining image acquisition parameters of a PCCT (photon-counting computed tomography) image acquisition device for acquiring PCCT images comprises: acquiring a plurality of candidate PCCT images using varying image acquisition parameters; presenting the plurality of candidate PCCT images to a user; receiving input from the user selecting one of the plurality of candidate PCCT images; and determining the image acquisition parameters as parameters corresponding to the selected candidate PCCT image.

Illustrative embodiment 3. The computer-implemented method of any one of illustrative embodiments 1-2, wherein determining image acquisition parameters of a PCCT (photon-counting computed tomography) image acquisition device for acquiring PCCT images comprises: acquiring a plurality of candidate PCCT images using varying image acquisition parameters; identifying one of the plurality of candidate PCCT images as having a highest analytical accuracy for performing the one or more medical imaging analysis tasks using the one or more machine learning based models; and determining the image acquisition parameters as parameters corresponding to the identified candidate PCCT image.

Illustrative embodiment 4. The computer-implemented method of any one of illustrative embodiments 1-3, wherein determining image acquisition parameters of a PCCT (photon-counting computed tomography) image acquisition device for acquiring PCCT images comprises: determining the image acquisition parameters of the PCCT image acquisition device for acquiring PCCT images optimized for performing the one or more medical imaging analysis tasks.

Illustrative embodiment 5. The computer-implemented method of any one of illustrative embodiments 1-4, wherein the image acquisition parameters comprise a number of energy bands and associated energy thresholds.

Illustrative embodiment 6. The computer-implemented method of any one of illustrative embodiments 1-5, wherein the image acquisition parameters comprise at least one of reconstructed image spacing, slice thickness, reconstruction kernels, or dose.

Illustrative embodiment 7. The computer-implemented method of any one of illustrative embodiments 1-6, wherein the one or more medical imaging analysis tasks comprise at least one of detection, segmentation, size quantification, typology classification, or malignancy assessment of the anatomical object of the patient.

Illustrative embodiment 8. The computer-implemented method of any one of illustrative embodiments 1-7, wherein the one or more machine learning based models are trained using annotated PCCT training images.

Illustrative embodiment 9. The computer-implemented method of any one of illustrative embodiments 1-8, wherein the anatomical object comprises a pulmonary nodule of the patient.

Illustrative embodiment 10. An apparatus comprising: means for determining image acquisition parameters of a PCCT (photon-counting computed tomography) image acquisition device for acquiring PCCT images; means for receiving one or more PCCT images of an anatomical object of a patient acquired using the PCCT image acquisition device configured with the image acquisition parameters; means for performing one or more medical imaging analysis tasks analyzing the anatomical object based on the one or more PCCT images using one or more machine learning based models; and means for outputting results of the one or more medical imaging analysis tasks.

Illustrative embodiment 11. The apparatus of illustrative embodiment 10, wherein the means for determining image acquisition parameters of a PCCT (photon-counting computed tomography) image acquisition device for acquiring PCCT images comprises: means for acquiring a plurality of candidate PCCT images using varying image acquisition parameters; means for presenting the plurality of candidate PCCT images to a user; means for receiving input from the user selecting one of the plurality of candidate PCCT images; and means for determining the image acquisition parameters as parameters corresponding to the selected candidate PCCT image.

Illustrative embodiment 12. The apparatus of any one of illustrative embodiments 10-11, wherein the means for determining image acquisition parameters of a PCCT (photon-counting computed tomography) image acquisition device for acquiring PCCT images comprises: means for acquiring a plurality of candidate PCCT images using varying image acquisition parameters; means for identifying one of the plurality of candidate PCCT images as having a highest analytical accuracy for performing the one or more medical imaging analysis tasks using the one or more machine learning based models; and means for determining the image acquisition parameters as parameters corresponding to the identified candidate PCCT image.

Illustrative embodiment 13. The apparatus of any one of illustrative embodiments 10-12, wherein the means for determining image acquisition parameters of a PCCT (photon-counting computed tomography) image acquisition device for acquiring PCCT images comprises: means for determining the image acquisition parameters of the PCCT image acquisition device for acquiring PCCT images optimized for performing the one or more medical imaging analysis tasks.

Illustrative embodiment 14. The apparatus of any one of illustrative embodiments 10-13, wherein the image acquisition parameters comprise a number of energy bands and associated energy thresholds.

Illustrative embodiment 15. A non-transitory computer-readable storage medium comprising instructions which, when executed by a computer, cause the computer to carry out operations comprising: determining image acquisition parameters of a PCCT (photon-counting computed tomography) image acquisition device for acquiring PCCT images; receiving one or more PCCT images of an anatomical object of a patient acquired using the PCCT image acquisition device configured with the image acquisition parameters; performing one or more medical imaging analysis tasks analyzing the anatomical object based on the one or more PCCT images using one or more machine learning based models; and outputting results of the one or more medical imaging analysis tasks.

Illustrative embodiment 16. The non-transitory computer-readable storage medium of illustrative embodiment 15, wherein determining image acquisition parameters of a PCCT (photon-counting computed tomography) image acquisition device for acquiring PCCT images comprises: acquiring a plurality of candidate PCCT images using varying image acquisition parameters; presenting the plurality of candidate PCCT images to a user; receiving input from the user selecting one of the plurality of candidate PCCT images; and determining the image acquisition parameters as parameters corresponding to the selected candidate PCCT image.

Illustrative embodiment 17. The non-transitory computer-readable storage medium of any one of illustrative embodiments 15-16, wherein the image acquisition parameters comprise at least one of reconstructed image spacing, slice thickness, reconstruction kernels, or dose.

Illustrative embodiment 18. The non-transitory computer-readable storage medium of any one of illustrative embodiments 15-17, wherein the one or more medical imaging analysis tasks comprise at least one of detection, segmentation, size quantification, typology classification, or malignancy assessment of the anatomical object of the patient.

Illustrative embodiment 19. The non-transitory computer-readable storage medium of any one of illustrative embodiments 15-18, wherein the one or more machine learning based models are trained using annotated PCCT training images.

Illustrative embodiment 20. The non-transitory computer-readable storage medium of any one of illustrative embodiments 15-19, wherein the anatomical object comprises a pulmonary nodule of the patient.

**Claims**

**1.** A computer-implemented method comprising:

determining (202) image acquisition parameters of a photon-counting computed tomography, PCCT, image acquisition device (102) for acquiring PCCT images;
receiving (204) one or more PCCT images (106) of an anatomical object of a patient acquired using the PCCT image acquisition device configured with the image acquisition parameters;
performing (206) one or more medical imaging analysis tasks analyzing the anatomical object based on the one or more PCCT images using one or more machine learning based models; and
outputting (208) results (110) of the one or more medical imaging analysis tasks.

2. The computer-implemented method of claim 1, wherein determining image acquisition parameters of a photon-counting computed tomography, PCCT, image acquisition device for acquiring PCCT images comprises:

   acquiring a plurality of candidate PCCT images using varying image acquisition parameters;
   presenting the plurality of candidate PCCT images to a user;
   receiving input from the user selecting one of the plurality of candidate PCCT images; and
   determining the image acquisition parameters as parameters corresponding to the selected candidate PCCT image.

3. The computer-implemented method of claim 1 or 2, wherein determining image acquisition parameters of a PCCT (photon-counting computed tomography) image acquisition device for acquiring PCCT images comprises:

   acquiring a plurality of candidate PCCT images using varying image acquisition parameters;
   identifying one of the plurality of candidate PCCT images as having a highest analytical accuracy for performing the one or more medical imaging analysis tasks using the one or more machine learning based models; and
   determining the image acquisition parameters as parameters corresponding to the identified candidate PCCT image.

4. The computer-implemented method of any one of claims 1 - 3, wherein determining image acquisition parameters of a PCCT (photon-counting computed tomography) image acquisition device for acquiring PCCT images comprises:

   determining the image acquisition parameters of the PCCT image acquisition device for acquiring PCCT images optimized for performing the one or more medical imaging analysis tasks.

5. The computer-implemented method of any one of claims 1 - 4, wherein the image acquisition parameters comprise a number of energy bands and associated energy thresholds.

6. The computer-implemented method of any one of claims 1 - 5, wherein the image acquisition parameters comprise at least one of reconstructed image spacing, slice thickness, reconstruction kernels, or dose.

7. The computer-implemented method of any one of claims 1 - 6, wherein the one or more medical imaging analysis tasks comprise at least one of detection, segmentation, size quantification, typology classification, or malignancy assessment of the anatomical object of the patient.

8. The computer-implemented method of any one of claims 1 - 7, wherein the one or more machine learning based models are trained using annotated PCCT training images.

9. The computer-implemented method of any one of claims 1 - 8, wherein the anatomical object comprises a pulmonary nodule of the patient.

10. The computer-implemented method of any one of claims 1 - 9, further comprising acquiring one or more PCCT images (106) of an anatomical object of a patient using the PCCT image acquisition device (714) configured with the image acquisition parameters.

11. An apparatus comprising:

   means for determining (202) image acquisition parameters of a photon-counting computed tomography, PCCT, image acquisition device (102) for acquiring PCCT images;
   means for receiving (204) one or more PCCT images (106) of an anatomical object of a patient acquired using the PCCT image acquisition device configured with the image acquisition parameters;

means for performing (206) one or more medical imaging analysis tasks analyzing the anatomical object based on the one or more PCCT images using one or more machine learning based models; and

means for outputting (208) results (110) of the one or more medical imaging analysis tasks.

12. The apparatus of claim 11, further comprising a photon-counting computed tomography, PCCT, image acquisition device (102) for acquiring PCCT images.

13. The apparatus of claim 11 or 12, wherein the means for determining image acquisition parameters of a photon-counting computed tomography, PCCT, image acquisition device for acquiring PCCT images comprises means for performing the method steps of any one of claims 2 - 4.

14. The apparatus of any one of claims 11 - 13, wherein the means for determining (202) image acquisition parameters, the means for receiving (204) one or more PCCT images (106), the means for performing (206) one or more medical imaging analysis tasks, and the means for outputting (208) are configured to cooperatively carry out the method of any one of claims 1 - 10.

15. A non-transitory computer-readable storage medium comprising instructions which, when executed by a computer, cause the computer to carry out operations comprising the steps according to the method of any one of claims 1 - 10.

FIG. 1

EP 4 631 435 A1

# FIG. 2

200

```
┌─────────────────────────────────────────────────────────┐
│  Determine image acquisition parameters of a PCCT image  │
│  acquisition device for acquiring PCCT images            │
│                         202                              │
└─────────────────────────────────────────────────────────┘
```

```
┌─────────────────────────────────────────────────────────┐
│  Receive one or more PCCT images of an anatomical object │
│  of a patient acquired using the PCCT image acquisition  │
│  device configured with the image acquisition parameters │
│                         204                              │
└─────────────────────────────────────────────────────────┘
```

```
┌─────────────────────────────────────────────────────────┐
│  Perform one or more medical imaging analysis tasks      │
│  analyzing the anatomical object based on the one or more│
│  PCCT images using one or more machine learning based    │
│  models                                                  │
│                         206                              │
└─────────────────────────────────────────────────────────┘
```

```
┌─────────────────────────────────────────────────────────┐
│  Output results of the one or more medical imaging       │
│  analysis tasks                                          │
│                         208                              │
└─────────────────────────────────────────────────────────┘
```

FIG. 3

FIG. 4

FIG. 5

**FIG. 6**

EP 4 631 435 A1

FIG. 7

Europäisches
Patentamt

European
Patent Office

Office européen
des brevets

## EUROPEAN SEARCH REPORT

**Application Number**

EP 25 16 8148

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/233645 A1 (MORARD VINCENT JEROME [FR] ET AL) 29 July 2021 (2021-07-29) * abstract * * figures 1-11 * * paragraph [0004] - paragraph [0144] * ----- | 1-15 | INV. A61B6/03 A61B6/42 A61B6/00 |
| X | ZIMMERMAN KEVIN C ET AL: "Experimental investigation of neural network estimator and transfer learning techniques for K-edge spectral CT imaging", MEDICAL PHYSICS., [Online] vol. 47, no. 2, 6 January 2020 (2020-01-06), pages 541-551, XP093175903, US ISSN: 0094-2405, DOI: 10.1002/mp.13946 Retrieved from the Internet: URL:https://onlinelibrary.wiley.com/doi/full-xml/10.1002/mp.13946> [retrieved on 2025-07-28] * abstract * * figures 1-9 * * Sections 1 - 5 * ----- | 1,11,15 | |
| A | US 2023/320688 A1 (DATTA ARKA [US] ET AL) 12 October 2023 (2023-10-12) * abstract * * figures 1-11 * * paragraph [0004] - paragraph [0134] * ----- | 1-15 | **TECHNICAL FIELDS SEARCHED (IPC)** A61B |

The present search report has been drawn up for all claims

| Place of search | Date of completion of the search | Examiner |
|---|---|---|
| Munich | 29 July 2025 | Moehrs, Sascha |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 16 8148

This annex lists the patent family members relating to the patent documents cited in the above-mentioned European search report.
The members are as contained in the European Patent Office EDP file on
The European Patent Office is in no way liable for these particulars which are merely given for the purpose of information.

29-07-2025

| Patent document cited in search report | Publication date | Patent family member(s) | Publication date |
|---|---|---|---|
| US 2021233645 A1 | 29-07-2021 | CN 113240719 A<br>EP 3855391 A1<br>US 2021233645 A1 | 10-08-2021<br>28-07-2021<br>29-07-2021 |
| US 2023320688 A1 | 12-10-2023 | NONE | |

EPO FORM P0459

For more details about this annex : see Official Journal of the European Patent Office, No. 12/82